# EUROPEAN PATENT APPLICATION

(11) **EP 4 353 241 A1**
(43) Date of publication of application: **17.04.2024**
(21) Application number: 22820089.5
(22) Date of filing: 31.05.2022
(51) Int. Cl.: A61K 33/00, A61P 1/16, A61P 1/18, A61P 19/02, A61P 21/00, A61P 25/02, A61P 39/06

(54) **FINE SILICON PARTICLE-CONTAINING PREVENTIVE OR THERAPEUTIC AGENT FOR DISEASES**

(30) Priority: 11.06.2021 JP 2021097781
(71) Applicant: OSAKA UNIVERSITY, Suita-shi Osaka 565-0871 (JP)
(72) Inventor: SHIMADA, Shoichi, Suita-shi, Osaka 565-0871 (JP); KOYAMA, Yoshihisa, Suita-shi, Osaka 565-0871 (JP); KONDO, Makoto, Suita-shi, Osaka 565-0871 (JP); USUI, Noriyoshi, Suita-shi, Osaka 565-0871 (JP); KOBAYASHI, Hikaru, Suita-shi, Osaka 565-0871 (JP); KOBAYASHI, Yuki, Suita-shi, Osaka 565-0871 (JP)
(74) Representative: Zwicker, Jörk
(86) International application number: PCT/JP2022/022056
(87) International publication number: WO 2022/259907

(57) **Abstract**

[Object] An object is to provide a pharmaceutical or the like that can prevent or treat non-alcoholic fatty liver disease, pain associated with inactivity, or acute pancreatitis.

[Solving Means] When silicon small particles are orally administered or placed on a skin or a mucous membrane, non-alcoholic fatty liver disease, pain associated with inactivity, and acute pancreatitis can be prevented or treated. The silicon small particles are particles each of which generates hydrogen when brought into contact with an aqueous solution having a pH of 7 or more. Provided is a preventive or therapeutic agent, pharmaceutical composition, medical device, food, or beverage comprising the silicon small particles for non-alcoholic fatty liver disease, pain associated with inactivity, or acute pancreatitis. The silicon small particles are suitably silicon fine particles and/or aggregates of the silicon fine particles.

## Description

### Technical Field

The present invention relates to prevention or treatment of non-alcoholic fatty liver disease, pain associated with inactivity, and acute pancreatitis.

### Background Art

Non-alcoholic steatohepatitis (NASH) is hepatitis that occurs through accumulation of fat in the liver by a primary cause other than alcohol. NASH is caused by overeating or insufficient exercise, and is asymptomatic in most cases. A fatty liver, which is a pre-NASH stage, increases risks of arteriosclerosis, myocardial infarction, and stroke. When NASH continues, risks of developing liver cirrhosis and liver cancer are also increased. Accordingly, early treatment is important. Major treatment methods are dietary restriction and exercise therapy, and it is urgent to develop a therapeutic drug.

Excessive rest, bedriddenness, cast fixation as a conservative treatment method for a bone fracture, and other kinds of long-term immobility cause complications, such as disuse muscle atrophy, pain, nerve palsy, and cardiovascular disorder. Long-term immobility of a joint is considered to be a risk factor for an onset of intractable chronic pain such as complex regional pain syndrome (CRPS). A nonsteroidal antiinflammatory analgesic drug, acetaminophen, or the like is mainly used for pain treatment. However, chronic pain such as CRPS shows resistance to pharmaceutical agent treatment in some cases. Accordingly, there is a need for a therapeutic drug having a novel action and mechanism.

Acute pancreatitis is an acute inflammatory disease in which activated pancreatic enzymes autodigest the pancreas and surrounding organs. Acute pancreatitis is mainly caused by alcohol or gallstones, and exhibits symptoms, such as strong upper abdominal pain, vomiting, and fever. An increase in severity thereof may lead to consciousness disorder or a shock state. Severe acute pancreatitis is a highly lethal disease in which inflammatory substances such as cytokines produced by the autodigestion spread across a whole body through a bloodstream to cause shock, respiratory failure, or multiple organ failure including acute nephritis. A major treatment method for acute pancreatitis is nutritional management, such as fasting or infusion administration. As a therapeutic drug, there is known a protease inhibitor (gabexate mesilate), which suppresses activity of the pancreatic enzymes and prevents progression of pancreatitis. However, it is urgent to develop a radical treatment for acute pancreatitis.

It is known that reactive oxygen species are essential to life, but oxidize cells that make up a living organism to damage the cells. The reactive oxygen species include a superoxide anion radical, a hydroxyl radical, hydrogen peroxide, and singlet oxygen. It is known that the hydroxyl radical is a radical having extremely high oxidizing power, and when generated in a living organism, oxidizes a nearby living material, such as DNA, a lipid, or a protein, to damage an organ. The hydroxyl radical is said to cause, through such action, various diseases, such as cancer and lifestyle diseases, and aging.

Hydrogen is known as a substance that eliminates hydroxyl radicals produced in a body. It is water that is produced by hydrogen reacting with hydroxyl radicals, and hydrogen does not produce a substance harmful to a living organism. Accordingly, there are many reports on hydrogen water containing hydrogen, which eliminates hydroxyl radicals in the body.

However, a saturated hydrogen concentration at room temperature is 1.6 ppm, and an amount of hydrogen contained in 1 liter of hydrogen water even in a saturated state is a mere 18 milliliters (ml) in terms of gas. In addition, hydrogen has a small molecule size, and hence hydrogen in hydrogen water passes through a container to be diffused into air. Accordingly, it is difficult to maintain an amount of dissolved hydrogen in hydrogen water. In addition, even when hydrogen water having a high concentration is ingested, much of the hydrogen in the hydrogen water gasifies in the upper gastrointestinal tract such as the stomach, causing pneumophagia (so-called "burp") in some cases. Accordingly, by the method of ingesting hydrogen water, it is not easy to take, into the body, hydrogen in an amount enough for causing a reaction with hydroxyl radicals in the body. Further, even when hydrogen is absorbed and transferred to organs, its concentration returns to that before the ingestion of hydrogen water in about 1 hour. In addition, it is difficult to inhale gaseous hydrogen in daily life.

Silicon small particles can generate hydrogen through contact with water. This reaction hardly proceeds through contact with an acidic aqueous solution at a pH of less than 5, the reaction proceeds through contact with an aqueous solution at a pH of 7 or more, and the reaction proceeds faster at a pH of 8 or more. In addition, when the silicon small particles are subjected to surface treatment, the reactivity proceeds. Further, the silicon small particles continue generating hydrogen in a sustained manner over 20 hours or more while in contact with an aqueous solution, and under some conditions, 1 g of the silicon small particles generate 400 ml or more of hydrogen (Patent Literature 1, Patent Literature 2, and Non Patent Literature 1). 400 ml of hydrogen corresponds to hydrogen gas contained in 22 liters of saturated hydrogen water.

In Patent Literature 3, there is a description of a solid preparation having a capability of generating hydrogen, which contains silicon small particles as a main ingredient. However, there is no description that a disease can be prevented or treated with the silicon small particles.

In Patent Literature 4, there is a description of a hydrogen supply material including silicon small particles and a medium containing water. There is a description that the hydrogen supply material is used to supply hydrogen to a skin or a mucous membrane. However, there is no description that a disease can be prevented or treated with the silicon small particles.

In Patent Literature 5, there is a description of hydrogen peroxide aqueous solution treatment of silicon small particles. However, there is no description that a disease can be prevented or treated with the silicon small particles.

In Patent Literature 6, there is a description of a composition containing silicon small particles, and there is a mention of an aspect in which the particles are contained in a "base material", such as a medicine for an animal, food for livestock or a pet, feed for an animal, a medicine for a plant, fertilizer for a plant, or compost for a plant. There is a description: health promotion and/or disease prevention of an animal, but there is no description that the silicon small particles can prevent or treat a disease to such an extent as to be able to serve as a pharmaceutical.

In Patent Literature 7, there is mainly a description of a silicon oxide layer formed on a surface of each of silicon small particles. As a form of utilization, there are descriptions of feed, a supplement, a food additive, and dermal and/or transmucosal intake of hydrogen, and there is a description: health promotion and/or disease prevention of an animal. However, there is no description that the silicon small particles can prevent or treat a disease to such an extent as to be able to serve as a pharmaceutical.

With regard to a kidney disease, inflammatory bowel disease, arthritis, hepatitis, dermatitis, visceral discomfort, depression or a depressive state, Parkinson's disease, an autism spectrum disorder, memory impairment, spinal cord injury, hearing loss, cerebral ischemia-reperfusion injury, diabetes, and a hangover, the inventors of the present invention have found that the silicon small particles can prevent and treat those diseases, and have filed patent applications (Patent Literatures 8 to 22).

### Citation List

### Patent Literature

[PTL 1] JP 2016-155118 A
[PTL 2] JP 2017-104848 A
[PTL 3] WO 2017/130709 A1
[PTL 4] WO 2018/037752 A1
[PTL 5] WO 2018/037818 A1
[PTL 6] WO 2018/037819 A1
[PTL 7] WO 2019/211960 A1
[PTL 8] WO 2019/021769 A1
[PTL 9] WO 2019/235577 A1
[PTL 10] JP 2019-214556 A
[PTL 11] JP 2020-007300 A
[PTL 12] JP 2020-079228 A
[PTL 13] JP 2020-079240 A
[PTL 14] JP 2020-117480 A
[PTL 15] JP 2020-117481 A
[PTL 16] JP 2020-117482 A
[PTL 17] JP 2020-117483 A
[PTL 18] JP 2020-117484 A
[PTL 19] JP 2020-117485 A
[PTL 20] JP 2020-117486 A
[PTL 21] JP 2020-200302 A
[PTL 22] WO 2020/152985 A1

### Non Patent Literature

[NPL 1] Shinsuke Matsuda et al., Concentration of hydrogen molecules and splitting water using silicon nanoparticle, Extended Abstracts of The 62nd JSAP Spring Meeting, 2015, 11a-A27-6

### Summary of Invention

### Technical Problem

An object of the present invention is to provide a pharmaceutical, medical device, food, or beverage for prevention or treatment of non-alcoholic fatty liver disease, pain associated with inactivity, and acute pancreatitis.

### Solution to Problem

The inventors of the present invention have found that silicon small particles can prevent and/or treat non-alcoholic fatty liver disease, pain associated with inactivity, and acute pancreatitis. Thus, the inventors have completed the present invention.
1. A preventive or therapeutic agent for a disease, comprising silicon small particles, wherein the disease is at least one disease selected from the group consisting of: non-alcoholic fatty liver disease; pain associated with inactivity; and acute pancreatitis.
2. The preventive or therapeutic agent according to the above-mentioned item 1, wherein the disease is non-alcoholic fatty liver disease.
3. The preventive or therapeutic agent according to the above-mentioned item 2, wherein the non-alcoholic fatty liver disease is non-alcoholic steatohepatitis.
4. The preventive or therapeutic agent according to the above-mentioned item 1, the disease is pain associated with inactivity.
5. The preventive or therapeutic agent according to the above-mentioned item 4, wherein the pain associated with inactivity is pain associated with musculoskeletal disuse syndrome.
6. The preventive or therapeutic agent according to the above-mentioned item 5, wherein the pain associated with musculoskeletal disuse syndrome is pain associated with muscle atrophy or joint contracture.
7. The preventive or therapeutic agent according to the above-mentioned item 1, wherein the disease is acute pancreatitis.
8. The preventive or therapeutic agent according to the above-mentioned item 7, wherein the acute pancreatitis is severe acute pancreatitis.
9. The preventive or therapeutic agent according to any one of the above-mentioned items 1 to 8, wherein the silicon small particles are small particles each comprising simple substance of silicon capable of generating hydrogen through contact with water.
10. The preventive or therapeutic agent according to any one of the above-mentioned items 1 to 9, wherein the silicon small particles are silicon fine particles and/or aggregates of the silicon fine particles.
11. The preventive or therapeutic agent according to any one of the above-mentioned items 1 to 9, wherein the silicon small particles are porous silicon particles.
12. A pharmaceutical composition for preventing or treating a disease, comprising silicon small particles, wherein the disease is at least one disease selected from the group consisting of: non-alcoholic fatty liver disease; pain associated with inactivity; and acute pancreatitis.
13. A medical device for preventing or treating a disease, comprising silicon small particles, wherein the disease is at least one disease selected from the group consisting of: non-alcoholic fatty liver disease; pain associated with inactivity; and acute pancreatitis.
14. A food or beverage for preventing or treating a disease, comprising silicon small particles, wherein the disease is at least one disease selected from the group consisting of: non-alcoholic fatty liver disease; pain associated with inactivity; and acute pancreatitis.
15. A therapeutic agent for a disease, comprising silicon small particles, wherein the disease is at least one disease selected from the group consisting of: non-alcoholic fatty liver disease; pain associated with inactivity; and acute pancreatitis.
16. A method of preventing or treating a disease comprising administering silicon small particles, wherein the disease is at least one disease selected from the group consisting of: non-alcoholic fatty liver disease; pain associated with inactivity; and acute pancreatitis.
17. A method of treating a disease comprising administering silicon small particles, wherein the disease is at least one disease selected from the group consisting of: non-alcoholic fatty liver disease; pain associated with inactivity; and acute pancreatitis.
18. An agent for use in prevention or therapy of a disease, comprising silicon small particles, wherein the disease is at least one disease selected from the group consisting of: non-alcoholic fatty liver disease; pain associated with inactivity; and acute pancreatitis.
19. An agent for use in therapy of a disease, comprising silicon small particles, wherein the disease is at least one disease selected from the group consisting of: non-alcoholic fatty liver disease; pain associated with inactivity; and acute pancreatitis.
20. A use of silicon small particles for preparation of a preventive or therapeutic agent for at least one disease selected from the group consisting of: non-alcoholic fatty liver disease; pain associated with inactivity; and acute pancreatitis.
21. A use of silicon small particles for preparation of a therapeutic agent for at least one disease selected from the group consisting of: non-alcoholic fatty liver disease; pain associated with inactivity; and acute pancreatitis.

### Advantageous Effects of Invention

The preventive or therapeutic agent of the present invention can prevent and treat non-alcoholic fatty liver disease. The preventive or therapeutic agent of the present invention can prevent and treat hepatic dysfunction associated with non-alcoholic fatty liver disease. In addition, the preventive or therapeutic agent of the present invention can suppress the accumulation of fat in the liver. In addition, the preventive or therapeutic agent of the present invention can suppress liver fibrosis. The therapeutic agent of the present invention can suppress the exacerbation of a non-alcoholic fatty liver, and prevent non-alcoholic steatohepatitis.

The preventive or therapeutic agent of the present invention can prevent and treat pain associated with inactivity. In addition, the preventive or therapeutic agent of the present invention can prevent and treat pain associated with each of joint contracture and muscle atrophy.

The preventive or therapeutic agent of the present invention can prevent and treat acute pancreatitis. In addition, the preventive or therapeutic agent of the present invention can suppress loss of pancreatic acinar cells associated with acute pancreatitis, and can alleviate the infiltration of immune cells into a pancreas tissue.

Prevention and treatment with the preventive or therapeutic agent of the present invention can be a causal therapy for non-alcoholic fatty liver disease, pain associated with inactivity, and acute pancreatitis, and is excellent in effect and also excellent in safety. The excellence in safety enables long-term administration as well. Accordingly, the preventive or therapeutic agent of the present invention is greatly conducive to future medical care.

### Brief Description of Drawings

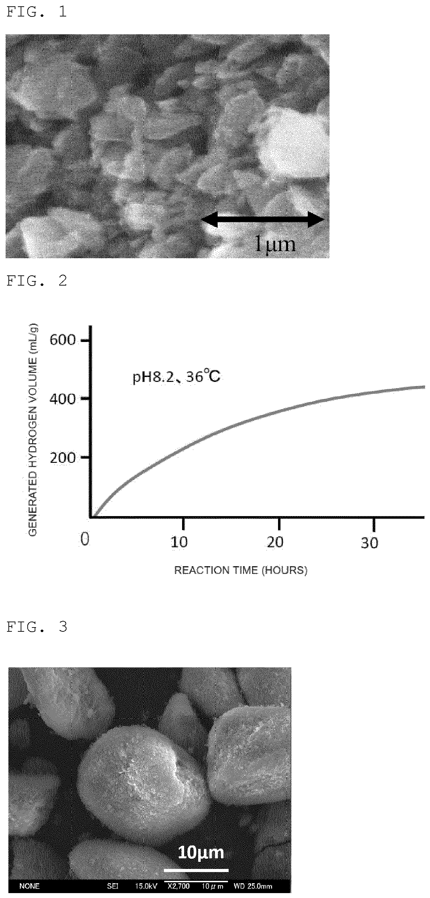

FIG. 1 is a photograph of silicon small particles (a mixture of silicon crystallites and aggregates thereof) taken with a scanning electron microscope (SEM) (Example 2).
FIG. 2 is a graph showing the volume (cumulative volume), per gram of silicon small particles, of hydrogen generated by bringing silicon small particles obtained in Example 2 into contact with water at 36°C and a pH of 8.2.
FIG. 3 is a photograph of silicon small particles (aggregates of silicon crystallites) taken with a scanning electron microscope (SEM) (Example 3).

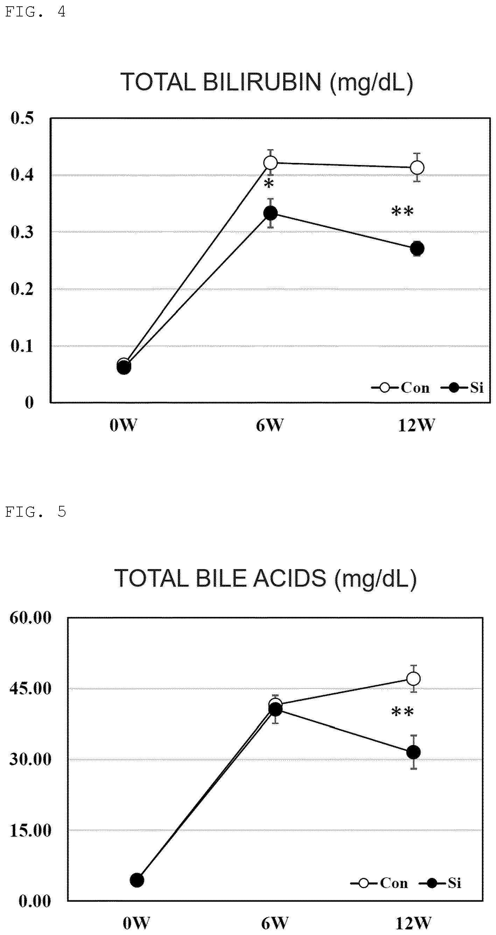

FIG. 4 shows the total bilirubin concentration in the blood of non-alcoholic steatohepatitis mouse models. It is shown that silicon small particle administration significantly reduced the total bilirubin concentration in the blood and alleviated hepatic dysfunction. Con: control group, Si: silicon small particle administration group, n=13 for each group, *p<0.05, **p<0.01, t-test (vs Con)
FIG. 5 shows the total bile acid concentration in the blood of non-alcoholic steatohepatitis mouse models. It is shown that silicon small particle administration significantly reduced the total bile acid concentration in the blood and alleviated hepatic dysfunction. Con: control group, Si: silicon small particle administration group, n=13 for each group, **p<0.01, t-test (vs Con)

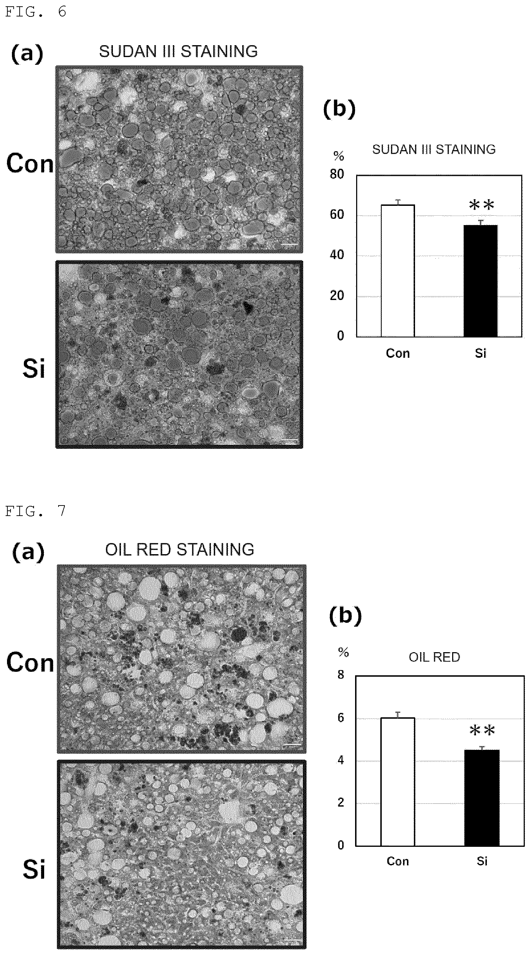

FIG. 6(a) includes Sudan III staining images of the livers of non-alcoholic steatohepatitis mouse models, and FIG. 6(b) is a graph showing the ratio of a stained area to the area of a whole tissue. Neutral fat is stained reddish orange. Silicon small particle administration significantly suppressed the accumulation of fat in the liver. Con: control group, Si: silicon small particle administration group, n=13 for each group, **p<0.01, t-test (vs Con)
FIG. 7(a) includes Oil Red staining images of the livers of non-alcoholic steatohepatitis mouse models, and FIG. 7(b) is a graph showing the ratio of a stained area to the area of a whole tissue. In Oil Red staining, only fat droplets of neutral fat are stained red. Silicon small particle administration significantly suppressed the accumulation of fat droplets in the liver. Con: control group, Si: silicon small particle administration group, n=13 for each group, **p<0.01, t-test (vs Con)

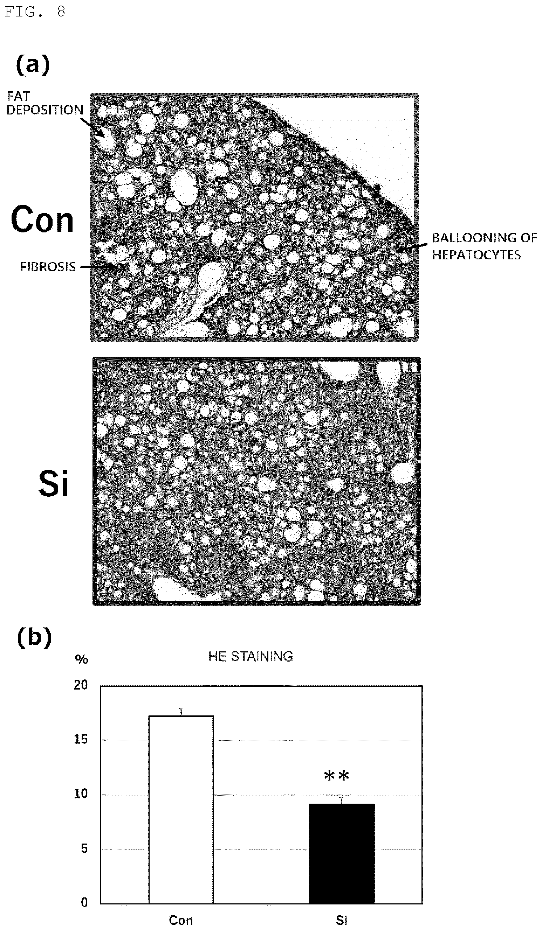

FIG. 8(a) includes HE staining images of the livers of non-alcoholic steatohepatitis mouse models, and FIG. 8(b) is a graph showing the degree of fat deposition (ratio of the area of vacuoles to the area of a whole tissue). In the images of FIG. 8 (a), fat droplets are observed as vacuoles. A flat white portion is not a vacuole but the vascular lumen. In the liver of a control mouse, ballooning and fibrosis of hepatocytes were observed. Meanwhile, those findings were not observed in the liver of a mouse administered silicon small particles. The silicon small particle administration significantly suppressed fat deposition. Con: control group, Si: silicon small particle administration group, n=13 for each group, **p<0.01, t-test (vs Con)

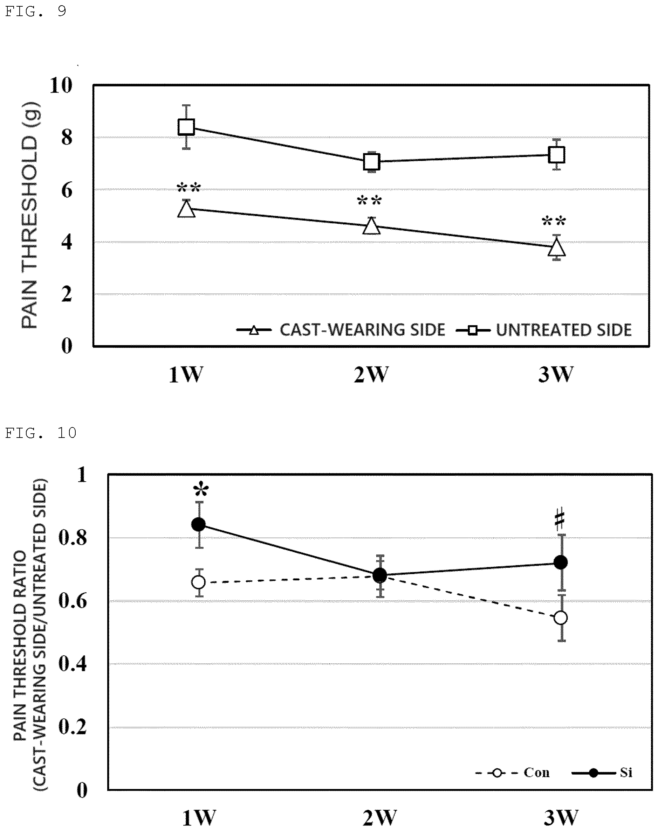

FIG. 9 is a graph showing the results of pain threshold measurement by a von Frey test of mouse models of disuse muscle atrophy by cast wearing (mice fed with normal feed). It was recognized that the cast wearing lowered the pain threshold. n=8-9, **p<0.01, t-test
FIG. 10 shows the results of pain threshold measurement by a von Frey test of cast wearing mouse models, and shows the ratio of the pain threshold of a cast-wearing side to the pain threshold of an untreated side. It is shown that the lowering of the pain threshold by the cast wearing was suppressed by silicon small particle administration. Con: control group, n=8-9, Si: silicon small particle administration group, n=10, #p<0.1, *p<0.05, t-test (vs Con)

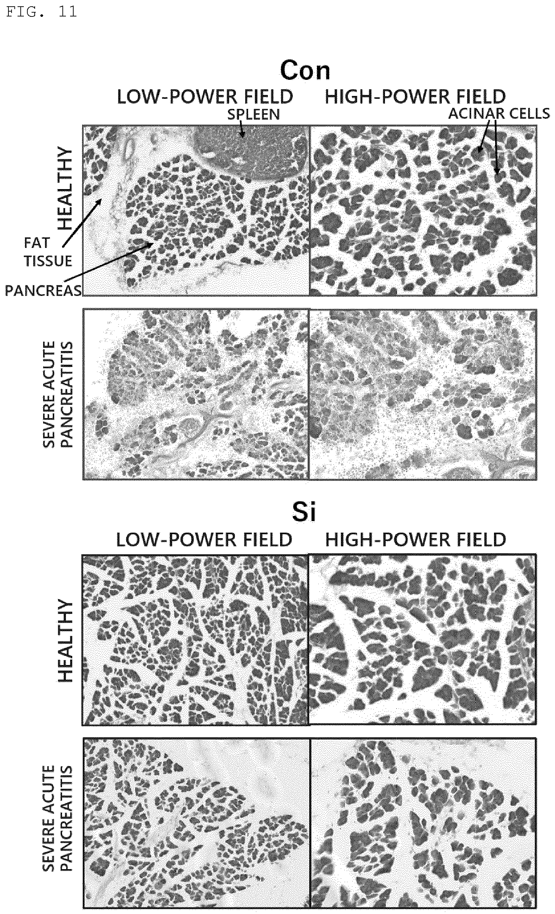

FIG. 11 includes HE staining images of the pancreas of a severe acute pancreatitis model, and shows the degree of inflammation of the pancreas. Low-power fields are shown on the left side, and high-power fields are shown on the right side. The infiltration of blood cells and the loss of acinar cells that perform exocrine secretion, which were observed in the pancreas of a control mouse, are hardly observed in the pancreas of a mouse administered silicon small particles, showing that the silicon small particle administration suppressed inflammation, and suppressed loss of pancreatic acinar cells. Con: control mice, Si: mice administered silicon small particles

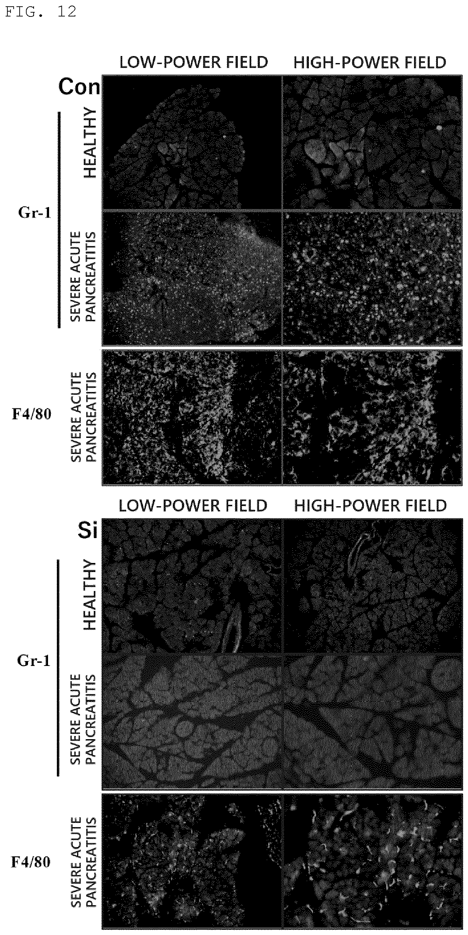

FIG. 12 includes immunofluorescence staining images of the pancreas of the severe acute pancreatitis model, and shows the degree of infiltration of neutrophils (marker: Gr-1) and the degree of infiltration of macrophages (marker: F4/80). Low-power fields are shown on the left side, and high-power fields are shown on the right side. In the pancreas of a control mouse, the infiltration of neutrophils and macrophages is remarkable, but in the pancreas of a mouse administered silicon small particles, the infiltration of neutrophils is not observed, with the infiltration of macrophages being observed extracellularly in a small number, showing that the silicon small particle administration suppressed the infiltration of immune cells. Con: control mice, Si: mice administered silicon small particles

### Description of Embodiments

Silicon small particles contained in a preventive or therapeutic agent of the present invention are small particles each comprising simple substance of silicon and capable of generating hydrogen through contact with water.

The "small particles each comprising simple substance of silicon capable of generating hydrogen through contact with water" (silicon small particles each having a capability of generating hydrogen) mean silicon small particles that generate hydrogen in a sustained manner when brought into contact with an aqueous solution at 36°C and a pH of 8.2, and that can generate 10 ml or more of hydrogen per gram of the silicon small particles in 24 hours. The volume of hydrogen to be generated is preferably 20 ml or more, 40 ml or more, 80 ml or more, 150 ml or more, 200 ml or more, or 300 ml or more.

The simple substance of silicon is high-purity silicon. Herein, the high-purity silicon refers to silicon having a purity of 98% or more, preferably 99% or more, more preferably 99.5% or more, still more preferably 99.9% or more, still more preferably 99.99% or more, still more preferably 99.999% or more.

The silicon small particles contained in the preventive or therapeutic agent of the present invention are preferably silicon fine particles, aggregates of the silicon fine particles, and/or porous silicon particles.

An active ingredient of the preventive or therapeutic agent of the present invention is preferably at least one kind of particles selected from the group consisting of: silicon fine particles; aggregates of the silicon fine particles; and porous silicon particles. That is, a preferred active ingredient may be the silicon fine particles alone, may be aggregates of the silicon fine particles alone, or may be the porous silicon particles alone. In addition, two or more kinds of silicon small particles may be contained as the active ingredient. The preventive agent or therapeutic agent of the present invention preferably comprises silicon fine particles and/or aggregates of the silicon fine particles. It is more preferred that aggregates of the silicon fine particles serve as a main ingredient.

When the simple substance of silicon is exposed to the atmosphere, its surface is oxidized to produce a silicon oxide layer. The silicon small particles in the present invention are preferably small particles each having a silicon oxide layer formed on the surface thereof. The silicon small particles in the present invention are preferably at least one kind of particles selected from the group consisting of: silicon fine particles each formed of simple substance of silicon, the fine particles each having a silicon oxide layer formed on the surface thereof; aggregates of the silicon fine particles; and porous particles each formed of simple substance of silicon, the porous silicon particles each having a silicon oxide layer formed on the surface thereof.

The content of silicon in each of the silicon small particles is preferably 10 wt% or more, more preferably 20 wt% or more, still more preferably 50 wt% or more, most preferably 70 wt% or more.

The silicon oxide layer is preferably a silicon oxide layer having added thereto hydroxy groups (-OH groups). The silicon oxide layer having added thereto hydroxy groups is a silicon oxide layer subjected to a treatment for increasing the number of hydroxy groups that the silicon oxide layer has. For example, hydroxy groups may be added to the silicon oxide layer by hydrophilic treatment. The silicon small particles each having formed thereon the silicon oxide layer having added thereto hydroxy groups are each improved in efficiency of contact between the surface and water, promoting the hydrogen generation reaction, and hence can generate a large volume of hydrogen. A method for the hydrophilic treatment is not particularly limited, and it is appropriate to use a known hydrophilic treatment method. Examples thereof include hydrogen peroxide aqueous solution treatment and nitric acid treatment. Of those, hydrogen peroxide aqueous solution treatment is preferred. The hydrogen peroxide aqueous solution treatment can remove the hydrogen of an SiH group of the silicon oxide layer on a particle surface and add a hydroxy group to the particle surface.

The silicon small particles each having formed the silicon oxide layer having added thereto hydroxy groups each preferably have, on the surface thereof, 5×10¹³ hydroxy groups/cm² or more, more preferably 1×10¹⁴ hydroxy groups/cm² or more, still more preferably 3×10¹⁴ hydroxy groups/cm² or more. The particle surface refers to the surface of each of the silicon fine particles, the surface of each of the porous silicon particles, the surface of each of the aggregates of the silicon fine particles, and the surface of each of the silicon fine particles for forming the aggregates.

As a specific method for the hydrogen peroxide aqueous solution treatment, for example, the silicon small particles are immersed and stirred in hydrogen peroxide aqueous solution. The concentration of hydrogen peroxide aqueous solution is preferably from 1% to 30%, more preferably from 1.5% to 20%, still more preferably from 2% to 15% or from 2.5% to 10%, most preferably from 3% to 5%. A period of time for which the silicon small particles are immersed and stirred is preferably from 5 minutes to 90 minutes, more preferably from 10 minutes to 80 minutes, still more preferably from 20 minutes to 70 minutes, most preferably from 30 minutes to 60 minutes. The hydrophilicity of each of the silicon small particles can be improved by treatment with hydrogen peroxide aqueous solution. However, when the treatment time is increased, the hydrogen generation reaction of each of the silicon small particles proceeds to increase the thickness of the oxide layer on each of the silicon small particles. The temperature of the hydrogen peroxide aqueous solution during the hydrogen peroxide aqueous solution treatment is preferably from 20°C to 60°C, more preferably from 25°C to 50°C, still more preferably from 30°C to 40°C, most preferably 35°C.

The shape of each of the silicon small particles is not limited. Examples thereof include an indefinite shape, a polygonal shape, a spherical shape, an oval shape, and a columnar shape.

The silicon small particles may be crystalline silicon small particles each having crystallinity. In addition, the silicon small particles may be amorphous silicon small particles having no crystallinity. When having crystallinity, the silicon small particles may be monocrystalline or polycrystalline. Of those, crystalline silicon small particles are preferred, and monocrystalline silicon small particles are more preferred.

The amorphous silicon small particles may be amorphous silicon small particles formed by a plasma CVD method, a laser ablation method, or the like.

The silicon oxide layer to be formed on the surface of each of the silicon small particles in the present invention may be a silicon oxide layer formed through natural oxidation by exposure to the atmosphere. In addition, the silicon oxide layer may be a silicon oxide layer artificially formed by a known method such as chemical oxidation with an oxidizing agent such as nitric acid.

The thickness of the silicon oxide layer only needs to be a thickness that stabilizes the small particles each formed of simple substance of silicon and enables efficient hydrogen generation. The thickness is, for example, from 0.3 nm to 5 nm, from 0.3 nm to 3 nm, from 0.5 nm to 2.5 nm, from 0.7 nm to 2 nm, from 0.8 nm to 1.8 nm, or from 1.0 nm to 1.7 nm. The silicon oxide layer may be a layer containing an oxide, such as Si₂O, SiO, Si₂O₃, or SiO₂. The oxide is generated by the bonding of silicon at the surface of each of the small particles formed of simple substance of silicon to oxygen. The oxide, such as Si₂O, SiO, or Si₂O₃, in which silicon has been incompletely oxidized promotes a hydrogen generation reaction.

The silicon fine particles may be crystalline silicon fine particles each having crystallinity. In addition, the silicon fine particles may be amorphous silicon fine particles having no crystallinity. When having crystallinity, the silicon fine particles may be monocrystalline or polycrystalline. The silicon fine particles are preferably crystalline silicon fine particles, more preferably monocrystalline silicon fine particles (hereinafter also referred to as "silicon crystallites").

The silicon fine particles in the present invention may be silicon fine particles each having a silicon oxide layer naturally or artificially formed after the production of the silicon fine particles. The silicon fine particles are more preferably fine particles each of which is a silicon crystallite having a silicon oxide layer formed on the surface thereof.

The silicon fine particles in the present invention may be particles obtained by grinding lumps of simple substance of silicon (high-purity silicon) or particles obtained by grinding particles of simple substance of silicon. When the silicon fine particles are produced by grinding lumps or particles of simple substance of silicon, the surface of each of the silicon fine particles is naturally oxidized, with the result that a silicon oxide layer is formed thereon.

The particle diameter of each of the silicon fine particles in the present invention (crystallite diameter when the fine particles are silicon crystallites) is preferably 0.5 nm or more and 100 um or less, more preferably 1 nm or more and 50 um or less, still more preferably 1.5 nm or more and 10 um or less, still more preferably 2 nm or more and 5 um or less, still more preferably 2.5 nm or more and 1 um or less, 5 nm or more and 500 nm or less, 7.5 nm or more and 200 nm or less, or 10 nm or more and 100 nm or less. When the particle diameter is 500 nm or less, a suitable hydrogen generation rate and generated hydrogen volume are obtained. When the particle diameter is 200 nm or less, a more suitable hydrogen generation rate and generated hydrogen volume are obtained.

The aggregates of the silicon fine particles in the present invention are aggregates of the above-mentioned silicon fine particles. The aggregates may be naturally formed, or may be artificially formed. Aggregates in which silicon fine particles each having a silicon oxide layer formed thereon are aggregated are preferred. It is conceived that naturally formed aggregates remain aggregated in the gastrointestinal tract. The aggregates each preferably have a structure in which internal voids are present and allow water molecules to infiltrate the aggregate to react with internal fine particles. The hydrogen generation rate of each of the naturally formed aggregates is not dependent on the size of the aggregate, and hence the aggregates each have a structure in which internal voids are present and allow water molecules to infiltrate the aggregate to react with internal fine particles.

The size of each of the aggregates of the silicon fine particles is not particularly limited. The particle diameter of each of the aggregates of the silicon fine particles is preferably 50 nm or more and 500 um or less.

The particle diameter of each of the silicon fine particles forming the aggregates of the silicon fine particles in the present invention is preferably 0.5 nm or more and 100 um or less, more preferably 1 nm or more and 50 um or less, still more preferably 1.5 nm or more and 10 um or less, still more preferably 2 nm or more and 5 um or less, still more preferably 2.5 nm or more and 1 µm or less, 5 nm or more and 500 nm or less, 7.5 nm or more and 200 nm or less, or 10 nm or more and 100 nm or less. The silicon fine particles forming the silicon aggregates may be crystalline silicon fine particles, or may be amorphous silicon fine particles. The aggregates are preferably aggregates of silicon crystallites each having a crystallite diameter of 1 nm or more and 10 um or less. Aggregates in which silicon crystallites each having a silicon oxide layer formed on the surface thereof are aggregated are preferred.

The preventive or therapeutic agent of the present invention comprises: silicon crystallites each preferably having a crystallite diameter of from 1 nm to 1 µm, more preferably having a crystallite diameter of 1 nm or more and 100 nm or less, the crystallites each having a silicon oxide layer formed on the surface thereof; and/or aggregates thereof. The preventive or therapeutic agent preferably comprises, as a main ingredient, aggregates of silicon crystallites each having a silicon oxide layer formed on the surface thereof.

The preventive or therapeutic agent of the present invention comprises: silicon crystallites each preferably having a crystallite diameter of from 1 nm to 1 µm, more preferably having a crystallite diameter of 1 nm or more and 100 nm or less, the crystallites each having formed on the surface thereof a silicon oxide layer having added thereto hydroxy groups; and/or aggregates thereof. The preventive or therapeutic agent preferably comprises, as a main ingredient, aggregates of silicon crystallites each having formed on the surface thereof a silicon oxide layer having added thereto hydroxy groups.

The porous silicon particles may be porous bodies of silicon particles. In addition, the porous silicon particles may be porous bodies obtained by aggregating and processing silicon fine particles. The porous silicon particles are preferably porous particles each formed of simple substance of silicon, the particles each having a silicon oxide layer formed on the surface thereof. The silicon oxide layer is more preferably a silicon oxide layer having added thereto hydroxy groups.

The porous silicon particles may be porous silicon particles each having crystallinity. In addition, the porous silicon particles may be amorphous porous silicon particles having no crystallinity. When having crystallinity, the porous silicon particles may be monocrystalline or polycrystalline.

The size of each of the voids present in the porous silicon particles is not limited, but may be generally from 0.3 nm to 1 µm, preferably from 0.5 nm to 0.1 um. The porous silicon particles each have a surface area sufficient for achieving a high capability of generating hydrogen. The porous silicon particles may be aggregated to form aggregates. The size of each of the porous silicon particles and the aggregates of the porous silicon particles is not particularly limited. The size may be preferably from 200 nm to 400 µm.

The aggregates of the silicon fine particles and the porous silicon particles are particles each having a large particle diameter as a whole and having a large surface area, and hence are particles suitable for oral administration. Large particles do not pass through the cell membranes and intercellular spaces of the gastrointestinal tract, in particular, the intestinal tract, and hence the silicon small particles are not absorbed in the body. Such particles are excellent from the viewpoint of safety.

In the preventive or therapeutic agent of the present invention, the particle size distribution of the silicon fine particles, the particle size distribution of the fine particles each formed of simple substance of silicon, or the size distribution of the crystallites is not particularly limited. The distribution may be polydisperse. The preventive or therapeutic agent may be a formulation containing silicon fine particles having particle sizes or crystallite sizes within a specific range. In addition, the size distribution of the aggregates of the silicon fine particles is not particularly limited.

The hydrogen generation rate may be adjusted on the basis of the particle diameters of the silicon small particles, their particle size distribution, and/or the thickness of the silicon oxide layer.

A production method for the silicon small particles of the present invention is not particularly limited, but the silicon small particles may be produced by physically grinding silicon-containing particles to target particle diameters. A suitable example of a physical grinding method is a bead mill grinding method, a planetary ball mill grinding method, a shock wave grinding method, a high-pressure collision method, a jet mill grinding method, or a grinding method in which two or more kinds of those methods are combined. In addition, a known chemical method may also be adopted. From the viewpoint of production cost or the ease of production control, the grinding method is suitably a physical grinding method. When small particles formed of fine particles of simple substance of silicon are exposed to the atmosphere, the surface is oxidized, with the result that a silicon oxide layer is formed thereon. In addition, the silicon oxide layer may be artificially formed, after the grinding, by a known method such as chemical oxidation with an oxidizing agent, such as hydrogen peroxide aqueous solution or nitric acid.

When produced by grinding the silicon-containing particles with a bead mill apparatus, the target sizes or particle size distribution of particles may be obtained by appropriately changing the size and/or kind of each of beads.

The silicon-containing particles serving as a starting material are not limited as long as the silicon-containing particles are high-purity silicon particles. An example thereof is commercially available powder of high-purity silicon particles. The silicon-containing particles serving as a starting material may be monocrystalline or polycrystalline, or may be amorphous.

The present application encompasses, an invention relating to a preventive or therapeutic agent for a disease comprising silicon small particles, an invention relating to a method of preventing or treating a disease comprising administering silicon small particles, an invention relating to an agent for use in prevention or therapy of a disease comprising silicon small particles, and an invention relating to a use of silicon small particles for preparation of a preventive or therapeutic agent for a disease, and the disease is at least one disease selected from the group consisting of: non-alcoholic fatty liver disease; pain associated with inactivity; and acute pancreatitis. The description, embodiments, and the like of the invention relating to the preventive or therapeutic agent for a disease comprising silicon small particles, in the description of the present application are the description, embodiments, and the like of all those inventions.

The preventive or therapeutic agent for at least one disease selected from the group consisting of: non-alcoholic fatty liver disease; pain associated with inactivity; and acute pancreatitis of the present invention encompasses an agent for preventing the disease, an agent for treating the disease, and an agent for preventing and treating the disease.

The prevention or treatment in the present invention encompasses, for example, the prevention of the onset of one or more symptoms of the disease, the delaying of the onset, the amelioration of the symptoms, the suppression of the exacerbation of the symptoms, the prevention of the recurrence of the symptoms, and early recovery from the symptoms.

The preventive or therapeutic agent of the present invention can prevent and treat non-alcoholic fatty liver disease. The non-alcoholic fatty liver disease in the present invention encompasses a non-alcoholic fatty liver, non-alcoholic steatohepatitis, and the like.

The preventive or therapeutic agent of the present invention can prevent and treat hepatic dysfunction associated with non-alcoholic fatty liver disease. In addition, the preventive or therapeutic agent of the present invention can suppress the accumulation of fat in the liver. In addition, the preventive or therapeutic agent of the present invention can suppress liver fibrosis. The therapeutic agent of the present invention can suppress the exacerbation of a non-alcoholic fatty liver, and prevent non-alcoholic steatohepatitis. It may also be said that the present invention is directed to an agent comprising silicon small particles for suppressing fat accumulation in the liver. It may also be said that the present invention is directed to an agent comprising silicon small particles for suppressing the exacerbation of a non-alcoholic fatty liver.

The preventive or therapeutic agent of the present invention can prevent and treat pain associated with inactivity. The pain associated with inactivity in the present invention encompasses pain that the inactivity itself causes and pain that occurs through the influence of the inactivity on a living organism. When inactivity is added to tissue damage associated with trauma or a regressive change associated with aging, pain occurs and develops into chronic pain in many cases. The pain associated with inactivity in the present invention encompasses systemic pain and regional pain.

The inactivity in the present invention refers to excessive rest, fixation of an affected part, non-loading, immobility, hypomotility, long-term recumbency, or the like. The inactivity itself causes pain, and besides, the inactivity is a risk factor for the onset of chronic pain. The inactivity in the present invention encompasses inactivity of part of the body (e.g., fixation of a limb) and inactivity of the whole body (e.g., rest). In addition, the inactivity in the present invention is not temporally limited, and encompasses short-term inactivity, such as short-term rest due to an acute disease and fixation of an affected part, and long-term inactivity, such as hypomotility due to an advanced age and long-term recumbency.

The pain associated with inactivity in the present invention encompasses pain after cast fixation, muscle pain associated with inactivity, for example, backache or shoulder stiffness associated with inactivity, and joint pain associated with inactivity.

Disuse syndrome refers to various conditions caused by inactivity. Musculoskeletal disuse syndrome in the present invention encompasses muscle atrophy, muscle weakness, joint contracture, bone atrophy, and the like. The preventive or therapeutic agent of the present invention can suitably prevent and treat pain associated with each of joint contracture, muscle atrophy, and muscle weakness, can more suitably prevent and treat pain associated with each of joint contracture and muscle atrophy, and can still more suitably prevent and treat pain associated with joint contracture.

The preventive or therapeutic agent of the present invention can prevent and treat acute pancreatitis. In particular, severe acute pancreatitis can be prevented and treated.

Acute pancreatitis is acute inflammation of the pancreas, and exhibits symptoms, such as strong upper abdominal pain, back pain, vomiting, and fever. Severe acute pancreatitis causes consciousness disorder, a shock state, respiratory failure, multiple organ failure including acute nephritis, and the like. The preventive or therapeutic agent of the present invention can prevent the manifestation of those symptoms, ameliorate the symptoms, and suppress the exacerbation of the symptoms.

The preventive or therapeutic agent of the present invention can suppress loss of pancreatic acinar cells associated with acute pancreatitis, and can suppress the infiltration of immune cells. It may also be said that the present invention is directed to an agent for suppressing loss of pancreatic acinar cells associated with acute pancreatitis.

The silicon small particles in the present invention each have a property of continuously generating hydrogen over a long period of time (20 hours or more) in vitro. The silicon small particles of the present invention generate hydrogen when brought into contact with an aqueous solution at a pH of 7 or more, and generate more hydrogen at a pH of 8 or more. Meanwhile, the silicon small particles each have a property of hardly generating hydrogen at a pH of 5 or less.

When the silicon small particles in the present invention are orally administered, the silicon small particles generate hydrogen in the intestines, though presumed to hardly generate hydrogen in the stomach, by virtue of such properties as described above. When the silicon small particles in the present invention were administered to normal mice, hydrogen generation was recognized in the cecum, part of the large intestine, and even when normal mice were given normal diet under the same conditions, hydrogen was below the detection limit. The retention time of food in the intestines is generally 20 hours or more in humans, and hence it is conceived that the preventive or therapeutic agent of the present invention, when orally administered, can keep generating hydrogen in the intestines over a long period of time, thereby supplying hydrogen to the body.

In addition, it is conceived that, when the silicon small particles are placed on a skin or a mucous membrane for a long period of time, hydrogen can be transdermally or transmucosally supplied to the body over a long period of time.

Unlike hydrogen water, the preventive or therapeutic agent of the present invention does not allow hydrogen to be diffused before its administration. This property contributes to maintaining the quality of products such as pharmaceuticals, and contributes to the convenience of manufacturers, sellers, and users.

It has been recognized through an investigation made by the inventors of the present invention that, when the silicon small particles according to the present invention were administered to rats and then the antioxidant power of plasma was evaluated (BAP test), the antioxidant power was significantly increased in the silicon small particle administration group (WO 2019/235577 A1).

A conceivable one of action mechanism by which non-alcoholic fatty liver disease, pain associated with inactivity, and acute pancreatitis are prevented and treated is as follows: the silicon small particles in the present invention keep generating hydrogen over a long period of time, the generated hydrogen is transferred into blood and to organs, and the hydrogen selectively reacts with a hydroxyl radical. In addition, in light of the improvement of the antioxidant power in blood, it is conceived that an antioxidant substance produced in the blood reacts with a hydroxyl radical. Further, it has been recognized through an investigation made by the inventors of the present invention that a remarkable effect is shown as compared to that of hydrogen water in an investigation involving using model animals of a disease associated with oxidative stress (WO 2019/235577 A1). Accordingly, it is conceived that a different action that hydrogen water does not have is obtained. A comparison between large intestine tissues of mice administered silicon small particles and non-administered mice found that the large intestine of the mice administered silicon small particles contained large amounts of glutathione monosulfide, cysteine monosulfide, and the like, which are involved in an antioxidant action in a living organism. This may be an action unique to the silicon small particles (JP 2020-117481 A, PCT/JP2021/016176). In addition, as another mechanism, for example, the following mechanism is conceivable: a protein containing a metal element such as cobalt that has captured hydrogen in an initial-stage generation state generated in the intestines through the reaction between the silicon small particles and water, or a protein having its reducing power enhanced as a result of a hydrogen atom donating an electron is transferred to organs, and reacts with and eliminates a hydroxy radical.

The preventive or therapeutic agent of the present invention may be used in combination with another therapeutic drug. A high therapeutic effect is expected through combined use with a pharmaceutical agent having a different action mechanism from that of the preventive or therapeutic agent of the present invention.

Targets of prevention or treatment with the preventive or therapeutic agent of the present invention are a human and a non-human animal. Preferred examples of the non-human animal include a pet and livestock.

One kind or two or more kinds of the silicon small particles in the present invention may be administered as they are to a human or a non-human animal, but as required, may be administered after having been mixed with an acceptable additive or carrier and formulated into a form well known to a person skilled in the art. Examples of such additive or carrier include a pH adjuster (e.g., sodium hydrogen carbonate, sodium carbonate, potassium carbonate, or citric acid), an excipient (e.g., a sugar derivative, such as mannitol or sorbitol; a starch derivative, such as corn starch or potato starch; or a cellulose derivative such as crystalline cellulose), a lubricant (e.g., a stearic acid metal salt such as magnesium stearate; or talc), a binder (e.g., hydroxypropyl cellulose, hydroxypropyl methyl cellulose, or polyvinylpyrrolidone), a disintegrant (e.g., a cellulose derivative, such as carboxymethyl cellulose or carboxymethylcellulose calcium), and an antiseptic (e.g., a p-oxybenzoic acid ester, such as methylparaben or propylparaben; or an alcohol, such as chlorobutanol or benzyl alcohol). Those additives and carriers may be blended alone or as a mixture thereof into the silicon small particles. A preferred example of the additive is a pH adjuster with which a pH can be adjusted to 8 or more. A preferred example of the pH adjuster is sodium hydrogen carbonate.

The administration route of the preventive or therapeutic agent of the present invention is not particularly limited, but a preferred example of the administration route is oral, transdermal, or transmucosal (e.g., the mouth, rectum, or vagina).

As a formulation for oral administration, there are given a tablet, a capsule, a granule, a powder, a syrup (dry syrup), an oral jelly, and the like. As a formulation for transdermal administration or for transmucosal administration, there are given a patch, an ointment, and the like.

The tablet, the capsule, the granule, the powder, and the like may each be turned into an enteric formulation. For example, the tablet, the granule, or the powder is subjected to enteric coating. As an enteric coating agent, an enteric coating agent that is poorly soluble in the stomach may be used. The capsule may be made enteric by filling an enteric capsule with the silicon small particles of the present invention.

The preventive or therapeutic agent of the present invention may be formulated into any of the above-mentioned dosage forms and then administered to a human or a non-human animal.

The content of the silicon small particles in the preventive or therapeutic agent of the present invention is not particularly limited, but is, for example, from 0.1 wt% to 100 wt%, from 1 wt% to 99 wt%, or from 5% to 95%.

The dose and number of times of administration of the silicon small particles in the present invention may be appropriately changed depending on conditions, such as the administration target, the age, body weight, and sex thereof, a purpose (e.g., for prevention or for treatment), the severity of a symptom, the dosage form, and the administration route. When the silicon small particles are administered to a human, a preferred dose thereof is, for example, as follows: about 10 mg to about 10 g, preferably about 100 mg to about 5 g, more preferably about 500 mg to about 2 g thereof are administered per day. In addition, the number of times of administration may be once or a plurality of times per day, or once every few days. For example, the number of times of administration may be from 1 to 3 times, 1 or 2 times, or once per day.

The preventive or therapeutic agent for at least one disease selected from the group consisting of: non-alcoholic fatty liver disease; pain associated with inactivity; and acute pancreatitis, comprising silicon small particles of the present invention may be utilized for a pharmaceutical, a quasi-drug, a medical device, a food, or a beverage.

The present application also relates to an invention of a pharmaceutical composition for preventing or treating a disease, comprising silicon small particles, wherein the disease is at least one disease selected from the group consisting of: non-alcoholic fatty liver disease; pain associated with inactivity; and acute pancreatitis. The pharmaceutical composition in the present invention also encompasses a composition having such a mild action as to qualify as a quasi-drug. Examples of embodiments of the pharmaceutical composition of the present invention may include the embodiments of the invention relating to a preventive or therapeutic agent described above.

The present application also relates to an invention of a medical device for preventing or treating a disease, comprising silicon small particles, wherein the disease is at least one disease selected from the group consisting of: non-alcoholic fatty liver disease; pain associated with inactivity; and acute pancreatitis. The medical device in the present invention refers to, for example, a tool or instrument intended for use in the treatment or prevention of a disease of a human or a non-human animal. An example of the medical device is a mask. When the mask of the present invention is worn, hydrogen can be directly supplied to the trachea or the lungs. In addition, another example of the medical device is an adhesive bandage.

The present application also relates to an invention of a food or beverage for preventing or treating a disease, comprising silicon small particles, wherein the disease is at least one disease selected from the group consisting of: non-alcoholic fatty liver disease; pain associated with inactivity; and acute pancreatitis. Preferred examples of the food or beverage of the present invention include a health food, a food with function claims, and a food for specified health use. The health food, the food with function claims, and the food for specified health use are each a food or beverage capable of preventing the disease, delaying the onset thereof, and/or preventing the recurrence thereof. The form of the food or beverage is not limited. Examples thereof include: a form of a mixture obtained by mixing the silicon small particles into an existing food or beverage; and a formulated form. Examples thereof include a tablet, a capsule, a powder, a granule, and a jelly.

Now, the present invention is more specifically described by way of Examples. However, the present invention is by no means limited thereto.

### Examples

### <Example 1>

High-purity silicon powder (manufactured by Osaka Titanium Technologies Co., Ltd., particle size distribution: <ϕ300 um (silicon particles each having a crystal grain diameter of more than 1 um), purity: 99.9%) was sieved to remove particles of 45 um or more. 200 g of the resultant silicon particles were dispersed in 4 liters (L) of 99.5 wt% ethanol, ϕ0.5 um zirconia beads (volume: 750 ml) were added, and the mixture was finely divided by performing grinding (one-stage grinding) at a rotation speed of 2,500 rpm for 4 hours using a bead mill apparatus (manufactured by Aimex Co., Ltd., horizontal continuous ready mill (model: RHM-08)).

The ethanol solution containing the finely divided silicon particles was separated from the beads by a separation slit arranged in a grinding chamber of the bead mill apparatus, and was then heated to a temperature from 30°C to 35°C with a vacuum evaporator. The ethanol was evaporated to give the finely divided silicon particles (crystallites).

The finely divided silicon particles (crystallites) obtained by the method described above had an average crystallite diameter ranging from 20 nm to 30 nm, and most of the crystallites formed aggregates. In addition, the crystallites were each covered with a silicon oxide layer, and the silicon oxide layer had a thickness of about 1 nm. The obtained mixture of the silicon crystallites each having a silicon oxide layer formed thereon and the aggregates thereof is silicon small particles serving as an active ingredient in one embodiment of the present invention.

### <Example 2>

The silicon crystallites and aggregates thereof obtained in Example 1 were mixed with hydrogen peroxide aqueous solution (3 wt%) in a glass container, and the contents were stirred at 35°C for 30 minutes. The silicon crystallites and aggregates thereof treated with hydrogen peroxide aqueous solution were subjected to solid-liquid separation treatment using a known centrifugal separator to remove the hydrogen peroxide aqueous solution. After that, the resultant silicon crystallites and aggregates thereof were further mixed with ethanol (99.5 wt%), and the mixture was thoroughly stirred. The silicon crystallites and aggregates thereof mixed with ethanol were subjected to solid-liquid separation treatment using a known centrifugal separator to remove the highly volatile ethanol, followed by sufficient drying. The resultant mixture of the silicon crystallites each having a silicon oxide layer formed thereon and aggregates thereof subjected to the hydrogen peroxide aqueous solution treatment are silicon small particles serving as an active ingredient in one embodiment of the present invention. A scanning electron microscope (SEM) photograph of the obtained silicon small particles is shown in FIG. 1. The hydrogen generation rate of the aggregates of the silicon crystallites obtained was not dependent on the sizes of the aggregates.

The generated hydrogen volume of the silicon small particles (silicon crystallites and aggregates thereof) obtained in Example 2 was measured. 10 mg of the silicon small particles were placed in a glass bottle having a volume of 100 ml (borosilicate glass, thickness: about 1 mm, Laboran Screw Tube Bottle manufactured by AS ONE Corporation). Water adjusted with sodium hydrogen carbonate to a pH of 8.2 was placed in the glass bottle, which was sealed under the temperature condition of a liquid temperature of 36°C, and a hydrogen concentration in the liquid in the glass bottle was measured. A portable dissolved hydrogen meter (manufactured by DKK-TOA Corporation, model: DH-35A) was used for the measurement of the hydrogen concentration. The generated hydrogen volume per gram of the silicon small particles is shown in FIG. 2.

### <Example 3>

By the same method as in Example 2, the silicon small particles (silicon crystallites and aggregates thereof) obtained in Example 1 were treated with hydrogen peroxide aqueous solution, and mixed and stirred with ethanol. The silicon small particles mixed with ethanol were dried using a spray dryer (ADL311S-A, manufactured by Yamato Scientific Co., Ltd.). The obtained aggregates of the silicon crystallites are silicon small particles serving as an active ingredient in one embodiment of the present invention. A scanning electron microscope (SEM) photograph of the obtained silicon small particles (aggregates of silicon crystallites) is shown in FIG. 3.

### <Example 4>

One-stage grinding was performed in the same manner as in Example 1. The cp0.5 um zirconia beads (volume: 750 ml) used for the one-stage grinding were automatically separated from the solution containing the silicon crystallites in the bead mill grinding chamber. To the resultant solution containing the silicon crystallites, 0.3 um zirconia beads (volume: 750 ml) were added, and the silicon crystallites were finely divided through further grinding at a rotation speed of 2,500 rpm for 4 hours (two-stage grinding).

The beads were separated from the solution containing the silicon crystallites as described above, and the resultant ethanol solution containing the silicon crystallites was heated to 40°C using a vacuum evaporator in the same manner as in Example 1. Ethanol was evaporated to give two-stage ground silicon crystallites. The thus two-stage ground silicon crystallites each having a silicon oxide layer formed thereon are also silicon small particles serving as an active ingredient in one embodiment of the present invention.

### <Example 5>

The mixture of the silicon crystallites each having a silicon oxide layer formed thereon and aggregates thereof subjected to the hydrogen peroxide aqueous solution treatment obtained in Example 2 was filled into a commercially available capsule No. 3 to provide a capsule formulation. This capsule formulation contains, as its main ingredient, the aggregates of the silicon crystallites each having a silicon oxide layer formed thereon subjected to the hydrogen peroxide aqueous solution treatment, and further contains the silicon crystallites each having a silicon oxide layer formed thereon subjected to the hydrogen peroxide aqueous solution treatment.

### <Test Examples>

### A. Pharmacological Test of Silicon Small Particles (Non-alcoholic Fatty Liver Disease)

### A1. Preparation of Non-Alcoholic Steatohepatitis Model

CDAHFD60 (choline-deficient, methionine-lowered 60 Kcal% fat diet (Oriental Yeast Co., Ltd.) was given to prepare non-alcoholic steatohepatitis mouse models (Matsumoto, M. et al. Int. J. Exp. Path. (2013), 94, 93-103).

### A2. Preparation of Silicon Small Particle-Containing Diet

CDAHFD60 was mixed with the silicon small particles (silicon crystallites and aggregates thereof) produced in Example 2 so that the amount of the particles was 2.5 wt%. Further, an aqueous citric acid solution (pH 4) was added in an amount of about 0.5 wt% with respect to the total amount of the silicon small particles and the feed, and the mixture was kneaded using a known kneading apparatus to give silicon small particle-containing high-fat diet.

### A3. Silicon Small Particle Administration

C57BL6/J mice (male, 5-week-old, Charles River Laboratories Japan, Inc.) were obtained. Two weeks after the obtainment were set as an acclimation period in which normal feed (manufactured by Oriental Yeast Co., Ltd., model number: AIN93M) was given. After that, the mice were reared for 3 months by giving the silicon small particle-containing high-fat diet obtained in the foregoing section A2 or control diet (CDAHFD60). Each group consisted of 13 mice (n=13). A blood examination was performed the next day after the initiation of silicon small particle administration (0 weeks), after 6 weeks, and after 12 weeks. In both groups, the values of AST and ALT were greatly increased, indicating the onset of a fatty liver or hepatitis, by 6 weeks.

### A4. Blood Examination

Total bilirubin concentration in blood is shown in FIG. 4, and total bile acid concentration therein is shown in FIG. 5. It was demonstrated that the silicon small particle administration significantly suppressed hepatic dysfunction associated with non-alcoholic fatty disease.

### A5. Morphological Analysis

### A5-1. Accumulation of Fat

After the blood examination at 12 weeks, liver tissues were collected, and tissue sections were prepared. The degree of fat accumulation in the liver was evaluated by two kinds of fat staining methods, i.e., Sudan III staining and Oil Red staining (both staining solutions from Muto Pure Chemicals Co., Ltd.). Image acquisition and analysis were performed using a Keyence microscope. The Sudan III staining is a staining method by which neutral fat is stained reddish orange, and the Oil Red staining is a staining method by which fat droplets of neutral fat are stained red. Staining images and graphs showing the ratios (%) of stained areas to the areas of the whole tissues are shown in FIGS. 6 and FIGS. 7. It was demonstrated that the silicon small particle administration suppressed the accumulation of fat in the liver.

### A5-2. Fat Deposition and Fibrosis

The liver tissue sections prepared in the foregoing section A5-1 were HE-stained, followed by observation of vacuoles, fibrosis, and the like. Fat droplets in hepatocytes are found as vacuoles in a paraffin-embedded specimen. FIGS. 8 show staining images and the ratio (%) of the area of vacuoles to the area of the whole tissue. A flat white portion is not a vacuole but the vascular lumen. In the liver of the control mouse, ballooning and fibrosis of hepatocytes were observed. Meanwhile, those findings were not observed in the liver of the mouse administered the silicon small particles. It was demonstrated that the silicon small particle administration suppressed macrovesicular fat deposition, and also suppressed fibrosis.

### B. Pharmacological Test of Silicon Small Particles (Pain Associated with Inactivity)

### B1. Preparation of Silicon Small Particle-Containing Diet

Normal feed (manufactured by Oriental Yeast Co., Ltd., model number: AIN93M) was mixed with the silicon small particles (silicon crystallites and aggregates thereof) produced in Example 2 so that the amount of the particles was 2.5 wt%. Further, an aqueous citric acid solution (pH 4) was added in an amount of about 0.5 wt% with respect to the total amount of the silicon small particles and the feed, and the mixture was kneaded using a known kneading apparatus to give silicon small particle-containing diet.

### B2. Preparation of Cast-wearing Disuse Muscle Atrophy mouse models

C57BL6/J mice (male, 7-week-old, Japan SLC) were obtained.
One hind leg of each of the mice was fixed in an extended state with a casting tape capable of hardening when dipped in water (Alcare Co., Ltd.) to prepare cast wearing mouse models.

The cast wearing model is widely used for an investigation of disuse muscle atrophy or joint contracture. In the cast wearing model, a joint is brought into a constantly extended state by cast wearing, which reflects the joint contracture condition of a human. In addition, although joint immobilization by cast wearing maintains gravity and innervation under a normal environment, remarkable skeletal muscle atrophy is induced merely by restricting the contractile activity of skeletal muscle by joint fixation. Accordingly, the cast wearing model accurately reflects a skeletal muscle atrophy condition at the time of inactivity observed in the daily life of a human (Yuuki Tomiga et al., Fukuoka University Review of Sports and Health Science, 45(2), 53-58 (2015)).

### B3. Silicon Small Particle Administration

From 1 week before the cast wearing (one-leg fixation treatment), mice to be administered the silicon small particles were given the silicon small particle-containing diet prepared in the foregoing section B1, and control mice were given the normal feed of the foregoing section B1.

### B4. Evaluation of Pain Threshold

A pain test with a mechanical stimulus (von Frey test, Dynamic Plantar Aesthesiometer (Catalog No. 37450; UGO BASILE, Varese, Italy)) was performed after 1 week, after 2 weeks, and after 3 weeks from the one-leg fixation treatment to evaluate the pain threshold of each mouse.

First, the pain thresholds of the hind leg on the cast-wearing side and the hind leg on the untreated side of the control mice were compared to each other, and a lowering of the pain threshold associated with the cast wearing was recognized (FIG. 9). Next, the ratio of the pain threshold of the cast-wearing side to the pain threshold of the untreated side in each of the mice administered the silicon small particles and the control mice was calculated. The results are shown in FIG. 10. Pain associated with long-term joint contracture due to cast wearing increases in some proportion to the length of the duration (load). At 1 week of cast wearing (acute phase), a lowering of the pain threshold occurred in the unadministered group (control group), but the lowering of the threshold was significantly alleviated in the silicon small particle administration group. However, as a result of 2 weeks of cast wearing, the load due to long-term contracture increased, and a lowering of the threshold was also observed in the silicon small particle administration group. After 3 weeks from the wearing, in the unadministered group, a further increase in pain and a further lowering of the threshold were observed, whereas the silicon small particle administration group showed such a tendency that the exacerbation of the pain due to long-term joint contracture was alleviated. It is conceived that the silicon small particles are effective for alleviating the rapid increase in pain in the acute phase after the wearing and the increase in pain due to sustained immobility. It was demonstrated that pain associated with joint contracture or disuse muscle atrophy was alleviated by the silicon small particle administration.

### C. Pharmacological Test of Silicon Small Particles (Acute Pancreatitis)

### C1. Preparation of Severe Acute Pancreatitis Mouse models

L-Arginine was intraperitonially administered to C57BL6/J mice (male, 7-week-old, Japan SLC) at 1.5 mg/g of body weight every hour three times to prepare severe acute pancreatitis mouse models (Kui B. et al. PLOS ONE. 2015 February 17; 10(2): e0117588) .

### C2. Silicon Small Particle Administration

From 1 week before the preparation of the severe acute pancreatitis mouse models, mice to be administered the silicon small particles were given the silicon small particle-containing diet prepared in the foregoing section B1, and control mice were given the normal feed of the foregoing section B1.

### C3. Morphological Analysis

After 3 days from the preparation of the model, perfusion fixation with a 4% paraformaldehyde fixative was performed, the pancreas was collected, and tissue sections were prepared. The degree of inflammation, the infiltration of blood cells, and changes in tissue structure were evaluated through HE staining (FIG. 11). When acute pancreatitis was developed, the infiltration of blood cells and the loss of acinar cells, which were observed in the control mouse group, were hardly observed in the group of mice administered the silicon small particles. The degree of infiltration of immune cells was evaluated by an immunostaining method using an antibody against a neutrophil marker Gr-1 (Thermo Fisher Scientific Inc.) and an antibody against a macrophage marker F4/80 (Abcam) (FIG. 12). As shown in FIG. 11 and FIG. 12, it was demonstrated that the inflammation of the pancreas and the infiltration of immune cells were significantly suppressed in the group of mice administered the silicon small particles as compared to the control mouse group.

The above-mentioned results demonstrated that the silicon small particles in the present invention exhibited high preventive effects and high therapeutic effects on non-alcoholic fatty liver disease, pain associated with inactivity, and acute pancreatitis.

### Industrial Applicability

The present invention can provide a causal therapy for non-alcoholic fatty liver disease, pain associated with inactivity, and acute pancreatitis, thereby being greatly conducive to future medicine and health promotion.

## Claims

1. A preventive or therapeutic agent for a disease, comprising silicon small particles, wherein the disease is at least one disease selected from the group consisting of: non-alcoholic fatty liver disease; pain associated with inactivity; and acute pancreatitis.

2. The preventive or therapeutic agent according to claim 1, wherein the disease is non-alcoholic fatty liver disease.

3. The preventive or therapeutic agent according to claim 2, wherein the non-alcoholic fatty liver disease is non-alcoholic steatohepatitis.

4. The preventive or therapeutic agent according to claim 1, wherein the disease is pain associated with inactivity.

5. The preventive or therapeutic agent according to claim 4, wherein the pain associated with inactivity is pain associated with musculoskeletal disuse syndrome.

6. The preventive or therapeutic agent according to claim 5, wherein the pain associated with musculoskeletal disuse syndrome is pain associated with muscle atrophy or joint contracture.

7. The preventive or therapeutic agent according to claim 1, wherein the disease is acute pancreatitis.

8. The preventive or therapeutic agent according to claim 7, wherein the acute pancreatitis is severe acute pancreatitis.

9. The preventive or therapeutic agent according to any one of claims 1 to 8, wherein the silicon small particles are small particles each comprising simple substance of silicon capable of generating hydrogen through contact with water.

10. The preventive or therapeutic agent according to any one of claims 1 to 9, wherein the silicon small particles are silicon fine particles and/or aggregates of the silicon fine particles.

11. The preventive or therapeutic agent according to any one of claims 1 to 9, wherein the silicon small particles are porous silicon particles.

12. A pharmaceutical composition for preventing or treating a disease, comprising silicon small particles, wherein the disease is at least one disease selected from the group consisting of: non-alcoholic fatty liver disease; pain associated with inactivity; and acute pancreatitis.

13. A medical device for preventing or treating a disease, comprising silicon small particles, wherein the disease is at least one disease selected from the group consisting of: non-alcoholic fatty liver disease; pain associated with inactivity; and acute pancreatitis.

14. A food or beverage for preventing or treating a disease, comprising silicon small particles, wherein the disease is at least one disease selected from the group consisting of: non-alcoholic fatty liver disease; pain associated with inactivity; and acute pancreatitis.

15. A therapeutic agent for a disease, comprising silicon small particles, wherein the disease is at least one disease selected from the group consisting of: non-alcoholic fatty liver disease; pain associated with inactivity; and acute pancreatitis.
